(19) **Europäisches Patentamt European Patent Office Office européen des brevets**

(11) **EP 3 326 593 A1**

(12) **EUROPEAN PATENT APPLICATION**
published in accordance with Art. 153(4) EPC

(43) Date of publication:
**30.05.2018 Bulletin 2018/22**

(51) Int Cl.:
***A61F 13/02*** *(2006.01)* ***A61L 17/00*** *(2006.01)*

(21) Application number: **16830420.2**

(22) Date of filing: **21.07.2016**

(86) International application number:
**PCT/JP2016/071398**

(87) International publication number:
**WO 2017/018315 (02.02.2017 Gazette 2017/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **24.07.2015 JP 2015147235**

(71) Applicant: **Nichiban Co. Ltd.**
**Tokyo 112-8663 (JP)**

(72) Inventor: **IKAI, Tomonori**
**Tokyo 112-8663 (JP)**

(74) Representative: **Schwabe - Sandmair - Marx**
**Patentanwälte Rechtsanwalt**
**Partnerschaft mbB**
**Joseph-Wild-Straße 20**
**81829 München (DE)**

(54) **SKIN-SUTURING TAPE OR REINFORCING TAPE USED AFTER SUTURING SKIN**

(57) Problem
To provide a skin closure tape or a reinforcing tape used after skin-suture.
Solution
A skin closure tape or a reinforcing tape used after skin-suture that includes a backing made of at least a nonwoven fabric and an adhesion layer disposed on a surface on one side of the backing. A plurality of resin stripes are disposed on a surface on another side or/and the surface on the one side of the backing. The plurality of the resin stripes extend in a longitudinal direction of the backing.

# F I G. 1

**Description**

Technical Field

The present invention relates to a skin closure tape or a reinforcing tape used after skin suture.

Background Art

Conventionally, for some medical tapes, a nonwoven fabric is employed as a base material since it has high skin followability and moisture permeability. However, since the nonwoven fabric has a easily extended feature through the application of a load, when the nonwoven fabric is used as a base material of a skin closure tape or a reinforcing tape used after skin suture where more tight fixation to wounds will be required, the nonwoven fabric has disadvantage since fixing force is to be insufficient.

Therefore, for a tape that solves the low fixing-force problem, for example, Patent Literature 1 has proposed a surgical wound closure tape that is constituted of a substrate body of a nonwoven fabric and includes a plurality of concavities disposed over one surface of the substrate body. The surgical wound closure tape with the plurality of concavities over the surface of its substrate body is excellent in coatability which can provide comfort on an area where the tape is attached, and in elasticity which can prevent wounds from being opened under any conditions.

As the method of making a knitted fabric or a woven fabric hard to extend, for example, Patent Literature 2 discloses a method for performing a resin treatment on the fabric with stretch properties in multiaxial directions.

Prior Art Documents

Patent Literature

Patent Literature 1: Japanese Patent Application Laid-open No. 62-243557
Patent Literature 2: Japanese Patent Application Laid-open No. 2004-033265

Summary of Invention

Problems to be solved by Invention

The surgical wound closure tape described in Patent Literature 1 has however a problem such that the tape extends in every direction, even by weak force.

Since the resin-treated fabric disclosed in Patent Literature 2, which has been treated for making the knitted fabric or the woven fabric hard to extend, has a problem where the fabric has poor skin followability because of difficulties of extending in all directions. Further, resin coating the entire surface of the nonwoven fabric will cause the moisture permeability to decrease.

Incidentally, the reinforcing tape used after skin suture is applied for fixing wounds after skin suture. On the other hand, the skin closure tape will be used also for joining and fixing skins, closing wounds such as cut wounds or punctured wounds that may not require sutures.

In many cases, the skin closure tape or the reinforcing tape used after skin suture is attached to wounds to be joined or incision scars of sutured wounds in an approximately right angle. Therefore, in order to fix the joined or sutured wounds so as not to open, the skin closure tape or the reinforcing tape used after skin suture needs to be characterized as that it should be as hard to extend as possible in a longitudinal direction of the tape. On the other hand, in order to reduce skin irritancies, the tape should have extendable properties to some extent in a shorter side direction of the tape, for improving followability of skin movements. Furthermore, the skin closure tape or the reinforcing tape used after skin suture is also required to have high moisture permeability from the viewpoint of preventing sweat and rash to skin on which the tape is attached.

Therefore, it is an object of the present invention to provide a skin closure tape or a reinforcing tape used after skin suture configured to meet the following requests: fixing wounds, having excellent followability to skin movements (hereinafter also referred to as "skin followability"), and enjoying low skin irritancies to stress, sweatiness and rashes to skin (hereinafter also referred to as "skin irritation") on which the tape is attached.

Solution to Problem

The present inventors have been making intensive studies in order to solve the above problem. As a result, it was found that by disposing a plurality of resin stripes extending in a longitudinal direction on the surface of a backing including a nonwoven fabric as a main body, the backing will be hard to extend in the longitudinal direction but allowing to extend in a shorter side direction because of the resin stripes disposed. The present invention has been achieved because of the inventors' discoveries where the above will make a tape adaptable for a skin closure tape or a reinforcing tape used after skin suture.

That is, the present invention relates to a skin closure tape or a reinforcing tape used after skin suture, the tape including a backing made of at least a nonwoven fabric and an adhesion layer disposed on one surface of the backing. The invention also relates to the tape where a plurality of resin stripes are disposed on a surface on another side or/and the surface on the one side of the backing, the plurality of the resin stripes extending in a longitudinal direction of the backing.

According to the present invention, the following embodiments are further provided.

(1) The skin closure tape or the reinforcing tape used after skin suture where the plurality of the resin stripes are stripes that have constant widths and are regularly arranged on the surface of the backing.

(2) The skin closure tape or the reinforcing tape used after skin suture where the plurality of the resin stripes have equal pitch intervals.

(3) The skin closure tape or the reinforcing tape used after skin suture where the pitch interval of the plurality of the resin stripes is 0.1 mm to 4.0 mm.

(4) The skin closure tape or the reinforcing tape used after skin suture where the resin stripes form a linear or approximately linear striped pattern.

(5) The skin closure tape or the reinforcing tape used after skin suture where the nonwoven fabric is a polyester nonwoven fabric.

(6) The skin closure tape or the reinforcing tape used after skin suture where the nonwoven fabric has a basis weight of 10 $g/m^2$ to 100 $g/m^2$.

(7) The skin closure tape or the reinforcing tape used after skin suture where self-backside adhesive force is 3.0 N/6 mm to 4.5 N/6 mm in self-backside adhesive force test where adhesive force is to be measured in that the adhesion layer of the tape is attached on a surface of a backing of a tape different from the tape.

(8) The skin closure tape or the reinforcing tape used after skin suture where the resin stripe is a resin stripe made of an ethylene-vinyl acetate copolymer.

(9) The skin closure tape or the reinforcing tape used after skin suture where an application amount of the resin stripe is an amount of 10 $g/m^2$ to 100 $g/m^2$ per area of the resin stripe.

(10) The skin closure tape or the reinforcing tape used after skin suture where the tape has a 5%-tensile-load in a longitudinal direction of 4.0 N/6 mm to 15.0 N/6 mm and a 5%-tensile-load in a shorter side direction of 0.1 N/6 mm to 3.0 N/6 mm.

(11) The skin closure tape or the reinforcing tape used after skin suture where the resin stripe is disposed on an adhesion layer side of the backing.

Advantageous Effects of Invention

The skin closure tape or the reinforcing tape used after skin suture of the present invention has difficulties in extending in the longitudinal direction, thus enabling to fix the joined or sutured wounds so as not to be opened. On the other hand, the tape is allowed to extend in the shorter side direction, thus enjoying the effect of excellent skin followability. In the skin closure tape or the reinforcing tape used after skin suture in the present invention, for example, elongation under the load of 2 N in the longitudinal direction is 0.1% GL to 2.7% GL (6 mm in width, 25 mm in chuck) while elongation under the load of 2 N in the shorter side direction is 20% GL to 70% GL (6 mm in width, 25 mm in chuck).

The skin closure tape or the reinforcing tape used after skin suture in the present invention is allowed to adjust an extension degree in the longitudinal direction and in the shorter side direction while adjusting the width, the arrangement, the pitch interval, and the like of the resin stripe disposed on the surface of the backing that constitutes the tape.

Furthermore, since the skin closure tape or the reinforcing tape used after skin suture in the present invention retains a part where the resin stripe is not disposed on the surface of the backing, moisture permeability can be kept. Accordingly, the skin closure tape or the reinforcing tape used after skin suture in the present invention has excellent skin followability and excellent moisture permeability, thereby enjoying the effect of low skin irritation.

Brief Description of Drawings

Fig. 1 is a plan view of a skin closure tape or a reinforcing tape used after skin suture as one embodiment of the present invention.
Fig. 2 is a cross-sectional view taken along a line A-A in Fig. 1.
Fig. 3 is a cross-sectional view of a tape as another embodiment of the present invention.
Fig. 4 is a cross-sectional view of a tape as another embodiment of the present invention.

Description of Embodiments

Throughout this description, the meaning of "fix" should be taken as that joined or sutured wounds to which the tape of the present invention is attached will be secured to an extent that those wounds do not open in daily life. Specifically, adhesive force of the tape relative to YUPO in a longitudinal direction is approximately 1.0 N/6 mm to 3.0 N/6 mm.

In this description, "elongation under the load of 2 N" typically means force to pull the skin together for doing a skin-suture. Specifically, elongation of the tape under the load of 2 N in the longitudinal direction is approximately 0.1% GL to 2.7% GL (6 mm in width, 25 mm in chuck). Elongation under the load of 2 N in the shorter side direction of the tape is approximately 20% GL to 70% GL (6 mm in width, 25 mm in chuck).

In the present invention, properties of the tape such as moisture permeability indicate features relating to a backing, resin stripes disposed on the surface of the backing, and a multilayer structure constituted of adhesion layers.

The skin closure tape or the reinforcing tape used after skin-suture of the present invention includes a backing made of at least a nonwoven fabric and an adhesion layer disposed on one surface of the backing, and the backing includes a plurality of resin stripes on the one surface of the backing and/or the other surface of the backing, the plurality of resin stripes extending in a longitudinal direction of the backing.

The following further describes the present invention with reference to drawings illustrating preferable aspects of the present invention.

[Resin Stripe]

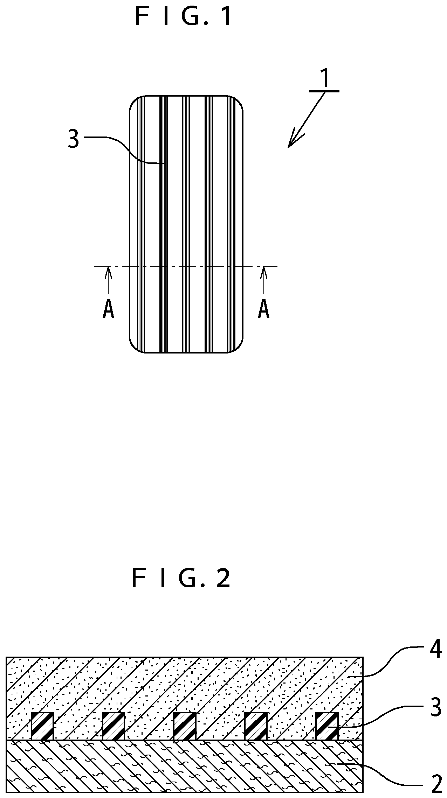

As illustrated in Fig. 1 and Fig. 2, in the present invention, a plurality of resin stripes 3 extending in a longitudinal direction are provided on one surface of the backing and/or the other surface of the backing. Since the plurality of the resin stripes 3 extending in the longitudinal direction are disposed on the surface of a backing 2, a tape 1 will be hard to extend in the longitudinal direction of the backing 2. Accordingly, the tape 1 can fix joined or sutured wounds so as not to open. In addition, since the tape 1 is allowed to extend to some extent in a shorter side direction because of the resin stripes disposed, this will improve the skin followability.

Specifically, the tape 1 of the present invention has one of the characteristics in that the plurality of resin stripes 3 extending in the longitudinal direction of the backing are disposed on the surface of the backing 2 constituting the tape. By adjusting widths, an arrangement, pitch intervals, and the like of the resin stripes, the extension degree of the tape can be adjusted in the longitudinal direction as well as in the shorter side direction.

In the present invention, the resin stripe should preferably have a certain width in order to more easily actualize the properties of: being hard to extend in the longitudinal direction to the extent that the joined or sutured wounds can be fixed so as not to open; and extending in the shorter side direction to the extent that the skin followability is improved.

Regarding the above width of the resin stripe, if the resin stripe has width too narrow, it makes the skin closure tape or the reinforcing tape used after skin-suture to become unnecessarily extendable in the longitudinal direction, possibly failing to firmly fix the joined or sutured wounds so as not to open. On the other hand, if the resin stripe has width too wide, it will cause the resin stripes to cover the most part of the surface of the backing, thereby making the skin closure tape or the reinforcing tape used after skin-suture less extendable in the longitudinal direction than required and less extendable also in the shorter side direction than necessary. Further, the moisture permeability of the tape may decrease.

Therefore, while being appropriately selected in consideration of the width of the backing, the width of the resin stripe should be, for example, 0.1 mm to 3.0 mm, preferably 0.15 mm to 2.5 mm, more preferably 0.2 mm to 2.0 mm, and especially preferably 0.3 mm to 1.0 mm. In the case that the resin stripe is less than 0.1 mm, as described above, the tape may fail to firmly fix the wounds. When the resin stripe exceeds 3.0 mm, as stated, extension in the longitudinal direction will become excessively reduced while extension in the shorter side direction becomes also reduced. Thus, the moisture permeability of the tape may decrease.

Particularly, in the present invention, since the plurality of the resin stripes 3 extendable in the longitudinal direction are disposed on the surface of the backing, this will enable the tape to become hard to extend in the longitudinal direction of the backing but yet extendable in the shorter side direction. To achieve this, in the present invention, the resin stripe should have the width in the above range and more preferably be regularly arranged on the surface of the backing.

Moreover, preferably the resin stripes should be arranged on the surface of the backing at equal pitch intervals. The pitch interval means a width (a length) of a part where the resin is not applied between each of the resin stripes. This enables each resin stripe to work uniformly on the backing, thus more easily actualizing the properties of: being hard to extend in the longitudinal direction to the extent that the joined or sutured wounds can be fixed so as not to open; and extending in the shorter side direction to the extent that the skin followability is improved.

Considering the pitch interval, if each of the pitch intervals is too narrow, it increases the number of the resin stripes arranged on the surface of the backing, resulting in that the most part of the surface of the backing would be covered thereby. This makes the skin closure tape or the reinforcing tape used after skin-suture less extendable in the longitudinal direction than required and less extendable also in the shorter side direction. In addition, the moisture permeability of the tape may decrease. On the other hand, if each of the pitch intervals is too wide, it decreases the number of the resin stripes arranged on the surface of the backing. This makes the skin closure tape or the reinforcing tape used after skin-suture extendable in the longitudinal direction than required, thus possibly failing to firmly fix the joined or sutured wounds so as not to open.

In consideration of the above, while appropriately selected depending on the width of the backing, the pitch interval should be, for example, 0.1 mm to 4.0 mm, preferably 0.15 mm to 3.0 mm, more preferably 0.2 mm to 2.0 mm, and especially preferably 0.3 mm to 1.0 mm. When the pitch interval is less than 0.1 mm, as explained, the number of the resin stripes will be excessive. Not only this causes extension of the tape in the longitudinal direction to be excessively reduced, but extension in the shorter side direction is also reduced. Further, the moisture permeability of the tape may decrease. When the pitch interval exceeds 4.0 mm, as described above, the number of the resin stripes will be not sufficient, making the tape possibly failed to firmly fix the wounds.

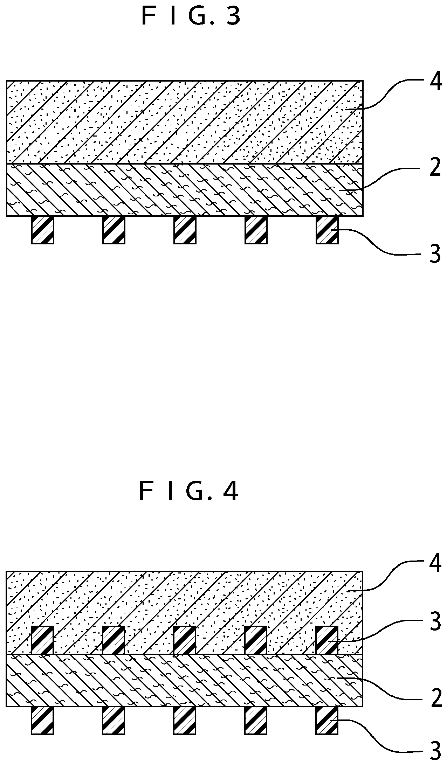

In the present invention, the resin stripes 3 may be disposed on a surface on an adhesion layer 4 side of the backing 2 as illustrated in Fig. 2, or the resin stripes 3 may be disposed on a surface of the backing 2 opposite to the adhesion layer 4 as illustrated in Fig. 3. Furthermore, the resin stripes 3 may be disposed on both the surface of the backing 2 on the adhesion layer 4 side and the surface of the backing 2 opposite to the adhesion layer 4 as illustrated in Fig. 4. However, the resin stripes 3 are preferably disposed on the surface of the backing 2 of the adhesion layer 4 side as illustrated in Fig. 2. Regarding the tape of the present invention, in the configuration where the resin stripes 3 are disposed on the surface of the backing 2 of the adhesion layer 4 side, the resin stripes 3 would be close to the wound part compared with the configuration where the resin stripes 3 are disposed on the surface of the backing 2 opposite to the side of the adhesion layer 4. Accordingly, effects to fix the joined or sutured wounds are greatly enhanced, and the skin followability will be notably improved. Degrees of penetration, tangling, adhesion, and the like of the adhesion constituting the adhesion layer 4 to the backing 2 are increased, thus more improving the anchoring property of the adhesion to the backing.

Although not limited, the resin stripes may be the ones preferably made of acrylic acid alkyl ester copolymer or made of ethylene-acetic acid copolymer.

The application amount of the resin stripe per area of the resin stripe is preferably 10 g/m$^2$ to 100 g/m$^2$, more preferably 15 g/m$^2$ to 70 g/m$^2$, and further preferably 20 g/m$^2$ to 50 g/m$^2$. In the case that the application amount of the resin stripe is less than 10 g/m$^2$, the amount of the resin stripe disposed on the surface of the backing would be too small for the resin stripe to sufficiently act upon the backing. This makes the skin closure tape or the reinforcing tape used after skin-suture extendable in the longitudinal direction than necessary, thereby possibly failing to firmly fix the joined or sutured wounds so as not to open. On the other hand, when the application amount of the resin stripe exceeds 100 g/m$^2$, the amount of the resin stripe disposed on the surface of the backing is too large. This makes the skin closure tape or the reinforcing tape used after skin-suture less extendable in the longitudinal direction than required while extension in the shorter side direction is also reduced. Accordingly, the moisture permeability of the tape may decrease.

In addition, the resin stripe is not limited to the one of a straight line, an approximately straight line, a wavy line, and a dashed line, but may be stripes of a zigzag-shaped linear or stripes of a straight line, an approximately straight line, a wavy line, and a dashed line, which of the stripes having uneven widths. Among them, it would be preferable for the resin stripes to have striped patterns for the following reasons. In the striped patterns of the straight line or the approximately straight line, the resin stripes can evenly act upon the backing, thereby more easily actualizing the properties of: being hard to extend in the longitudinal direction to the extent that the joined or sutured wounds can be firmly fixed so as not to open; and extending in the shorter side direction to the extent that the skin followability is improved.

[Backing]

The backing used in the present invention is the one which is constituted of at least nonwoven fabric. Accordingly, in the present invention, not only the backing constituted of only the nonwoven fabric, but also a backing where a film is laminated on the nonwoven fabric, a backing where a carrier film is disposed on the surface of the backing of the nonwoven fabric, and any similar backings may be employed.

As the nonwoven fabric, various kinds of fibers such as a natural fiber, a synthetic fiber, a recycled fiber, and an appropriate combination of these fibers may be employed, and specifically, an acrylic fiber nonwoven fabric, a nylon nonwoven fabric, a polypropylene nonwoven fabric, a polyamide nonwoven fabric, a polyester nonwoven fabric, a polyurethane nonwoven fabric, and similar nonwoven fabric may be employed. Especially, the polyester nonwoven fabric is preferable from the viewpoint that strength is maintainable easily when wet in water, and a self-backside adhesive force is much enhanced.

The nonwoven fabric normally has basis weight of 10 $g/m^2$ to 100 $g/m^2$, and from the viewpoint of further improving the moisture permeability of the tape and maintaining the strength of easy attachment, the basis weight is preferably 15 $g/m^2$ to 80 $g/m^2$, and more preferably 20 $g/m^2$ to 60 $g/m^2$. When the basis weight of the nonwoven fabric is less than 10 $g/m^2$, the stretch properties of the nonwoven fabric will be too high, and even if the width, the pitch interval, and the like of the resin stripe are adjusted, it may make difficult to actualize the following properties: being hard to extend in the longitudinal direction to the extent that the joined or sutured wounds can be firmly fixed so as not to open; and extending in the shorter side direction to the extent that the skin followability is improved. Additionally, the adhesion may exude through the surface of the nonwoven fabric where the adhesion is not applied, and the tape may decrease stiffness thus making it hard to be attached. When the basis weight of the nonwoven fabric exceeds 100 $g/m^2$, the stretch property of the nonwoven fabric will be too low. Then, it will make difficulty to adjust the extension of both in the longitudinal direction and in the shorter side direction of the resin stripe. Further, it may decrease the moisture permeability of the tape so as to increase the skin irritation and may cause an uncomfortable feeling when the tape is attached.

The carrier film may be the one publicly known. For instance, a glassine paper using a high-quality paper, a poly-laminated paper where a plastic film is laminated on a high-quality paper, and a film under a peeling treatment by which, for example, a silicone resin is applied over a surface of a plastic film are applicable.

While being able to properly set the thickness of the carrier film, the thickness may be generally equal to or more than 10 $\mu$m, and preferably equal to or more than 20 $\mu$m. The upper limit value of the thickness is normally 500 $\mu$m, but preferably 300 $\mu$m, and more preferably 200 $\mu$m.

[Adhesion Layer]

The adhesion layer constituting the tape of the present invention may be formed as a single adhesion layer, or may be formed by laminating a plurality of adhesion layers. When the adhesion layer is formed through the lamination of the plurality of adhesion layers, it is possible to laminate a plurality of different kinds of adhesion layers.

In the present invention, preferably, adhesion that forms the adhesion layer will be the one including at least one of adhesives selected from the group consisting of an acrylic adhesive, a rubber adhesive, an urethane adhesive, and a silicone adhesive. The adhesion may be used alone or as a mixture of two or more kinds. Among those adhesives, the acrylic adhesive is preferable, and the foamed acrylic adhesive will be further preferable from the viewpoint of sufficient adhesiveness to the skin surface, low irritancy to the skin, and high moisture permeability.

The acrylic adhesive includes, for example, an adhesive that contains a homopolymer of (meth)acrylic acid alkyl ester monomer such as butylacrylate, 2-ethylhexyl acrylate, and isononyl acrylate, or an acrylic copolymer where this (meth)acrylic acid alkyl ester monomer is copolymerized with one or more kinds of other copolymerizable monomers such as (meth)acrylic acid, vinyl acetate, styrene, vinylpyrrolidone, acrylamide, hydroxyethyl acrylate, and hydroxypropyl acrylate.

The adhesion layer may include a softener, a tackifier, a pH regulator, a medicinal ingredient, a filler, an antioxidant (an antioxidant agent, a preservative), a colorant, a perfume, and similar material, as an optional component.

Normally, the thickness of the adhesion layer is approximately 10 $\mu$m to 200 $\mu$m, and preferably 20 $\mu$m to 100 $\mu$m.

In the present invention, in order to protect the surface of the adhesion layer, a separation body may be disposed on the surface of the adhesion layer.

In the skin closure tape or the reinforcing tape used after skin-suture of the present invention, it can be defined as that a 5% tensile-load in the longitudinal direction is 4.0 N/6 mm to 15.0 N/6 mm, and a 5% tensile-load in the shorter side direction is 0.1 N/6 mm to 3.0 N/6 mm. Further, preferably the 5% tensile-load in the longitudinal direction is 4.5 N/6 mm to 13.0 N/6 mm, and the 5% tensile-load in the shorter side direction is 0.1 N/6 mm to 2.5 N/6 mm. Still further, more preferably the 5% tensile-load in the longitudinal direction is 5.0 N/6 mm to 11.0 N/6 mm, and the 5% tensile-load in the shorter side direction is 0.1 N/6 mm to 2.0 N/6 mm.

In the skin closure tape or the reinforcing tape used after skin-suture of the present invention, it can be defined as that elongation under the load of 2 N in the longitudinal direction is 0.1% GL to 2.7% GL (6 mm in width, 25 mm in chuck), and elongation under the load of 2 N in the shorter side direction is 20% GL to 70% GL (6 mm in width, 25 mm in chuck). Further, preferably elongation under the load of 2 N in the longitudinal direction is 0.1% GL to 2.0% GL (6 mm in width, 25 mm in chuck), and elongation under the load of 2 N in the shorter side direction is 30% GL to 70% GL (6 mm in width, 25 mm in chuck).

Moreover, in the skin closure tape or the reinforcing tape used after skin-suture of the present invention, according to a self-backside adhesive force test where an adhesive force is measured under a condition that the adhesion layer of the tape is attached on a surface of a backing of a tape different from the tape, a self-backside adhesive force is 3.0 N/6 mm to 4.5 N/6 mm, preferably 3.3 N/6 mm to 4.5 N/6 mm, and more preferably 3.5 N/6 mm to 4.5 N/6 mm,. Accordingly, the tape of the present invention has the adhesive force to the tape and is less likely to be separated even if the tape is attached on the surface of the tape. Then, in the present invention, a plurality of the tapes may be laminated superimposingly.

Especially, when the self-backside adhesive force is 3.5 N/6 mm to 4.5 N/6 mm, the tape of the present invention can be rigidly fixed onto the tape.

The tape of the present invention may be provided in a roll shape; however, it is more preferable to provide the tape in a sheet shape, that is, the tape of the present invention is to be laminated on a separation body whose area is larger than the one of the tape of the present invention. When the tape of the present invention is provided in the roll shape, the separation body is preferably laminated on the adhesion layer in a winding state.

The method of manufacturing the skin closure tape or the reinforcing tape used after skin-suture of the present invention is not limited to the particular one; however, the skin closure tape or the reinforcing tape used after skin-suture of the present invention may be manufactured through general manufacturing methods of a patch material.

The resin stripe may be formed by applying a resin such as acrylic acid alkyl ester copolymer or ethylene-acetic acid copolymer over the surface of the backing with known methods.

The adhesion layer may be formed by, for example, supplying the adhesion on a top surface of either the backing or the separation body with a method such as a coating method, an extrusion molding method and the like. Further, the adhesion layer may be formed by supplying the adhesion between the backing and the separation body. Still further, the backing and the adhesion layer may be formed by coextrusion molding.

From the viewpoint of easily producing an adhesive skin patch with stable quality, preferably, the method of manufacturing the adhesive skin patch includes a process of coating the adhesion over the top surface of the separation body to form the adhesion layer. Although not specifically limited, the coating method of the adhesion may include a spread coating, a hotmelt coating, an emulsion coating, or similar coating. Especially, in order to obtain a thin adhesion layer having a thickness equal to or less than 50 $\mu$m, the spread coating method is preferable. Specifically, while a preliminarily formed separation body travels in one direction, the adhesion in which to form the adhesion layer is coated over the top surface of the separation body. A solvent is then dried and removed, thereby forming the adhesion layer. Next, the adhesion layer is made to face the separation body, and the backing containing a plurality of the resin stripes is laminated so as to obtain the adhesive skin patch where the backing, the adhesion layer, and the separation body are laminated in this order.

Regarding the coating patterns of the adhesion layer, the entire surface of the backing may be coated, but the surface of the backing may be partially coated. When partially coated, any patterns such as a grid shape, a net shape, a granular shape, an arabesque shape, and similar shapes will be selectable. As discussed above, by partially disposing the adhesion layer on the one surface of the backing, it will ensure sufficient improvement of air permeability, moisture permeability, and similar properties and will ensure sufficient reduction of irritancy when separated from the skin.

[Embodiments]

The following will further describes the present invention with embodiments; however, the present invention is not limited thereto. Measuring methods for the properties of the skin closure tape or the reinforcing tape used after skin-suture can be defined as follows.

[Measurement of Adhesive Force to YUPO]

Under the atmosphere of temperature 23°C and 50% RH, the test piece of the tape is cut into 50 mm in length and 6 mm in width and attached onto YUPO (registered trademark). A 1 kg rubber roll was then reciprocated twice at a speed of 600 mm/minute to perform a pressure-bonding onto the test piece. Within one minute after the press-bonding, a peel force was measured under the condition of 180 degrees in peel angle and 300 mm/minute in peel speed, thus obtaining the adhesive force to YUPO of the test piece. The measurement was performed three times, and the average value was taken as the adhesive force to YUPO (unit: N/6 mm) of the tape.

When the adhesive force to YUPO of the tape is 1.0 N/6 mm to 3.0 N/6 mm, the tape obtains the adhesive force necessary for fixing.

[Measurement of Self-Backside Adhesive Force]

According to 180-degree peeling method specified in Japanese Industrial Standard Z-0237, the self-backside adhesive force of the tape was measured. Specifically, a tape (a test piece) cut out into 6 mm in width was placed in such a manner that its adhesion layer is placed on the underside, and the 1 kg rubber roll was reciprocated twice at the speed of 600 mm/minute to the back surface (a backing surface) of the identical tape for press-bonding. The free-margin portion of the test piece was folded back to 180 degrees and then measured a 180-degree peel adhesive force (the self-backside adhesive force) at the peel speed of 300 mm/minute, within one minute after the press-bonding. The measurement atmosphere was set to 23 $\pm$ 2°C in temperature and 50 $\pm$ 5% in relative humidity. If the self-backside adhesive force is equal to or more than 3.0 N/6 mm, it will reduce the following in the actual use of the tape: (1) the laminated portions between each tape are peeled off because of been rubbed against wearing clothing; and (2) tearing-off occurs at the end of the laminated portions between each tape.

[Measurement of 5%-Tensile-Load, Maximum Point Load, Elongation under Load of 2 N, and Elongation under Load of 3 N]

On the tapes of embodiments and comparative examples, tensile tests were performed according to Japanese Industrial Standard K-7113, thus measuring the 5%-tensile-loads. For the test piece of each tape, the piece was obtained by making a stripe cut out into 6 mm in width in the shorter side (CD) direction and cut out into 100 mm in length in the longer side (MD) direction. Cloth adhesive tapes <LS> No. 123 manufactured by NICHIBAN CO., LTD were adhered to both ends of the test piece in such a manner that the distance between chucks is 25 mm. In this manner, test pieces for measuring the tensile strength were prepared. The test pieces were then placed on the crosshead of a tensilon-type tensile tester at the grip distance of 25 mm. An upper crosshead was raised at a speed of 300 mm/minute to pull the test piece, thus performing the test to calculate the 5%-tensile-load, the maximum point load, the elongation under the load of 2 N, and the elongation under the load of 3 N.

The load where a pull length had reached 5% extension based on the original test piece as a reference was defined as the 5%-tensile-load. The unit of the tensile load was indicated by newton (N)/6 mm.

A point where the load had become maximum was defined as the maximum point load, and the unit was indicated by newton (N)/6 mm.

The elongation under the load of 2 N or 3 N was calculated to be defined as the elongation under the load of 2 N or the elongation under the load of 3 N, respectively. The unit of the elongation under the load was % GL.

Respective three test pieces cut out in the shorter side (CD) direction and the longer side (MD) direction of the tape were prepared, and the measurements were performed on all the three pieces. The average value of the three pieces was defined as the measurement value of the tensile load in each of the CD direction and the MD direction. Measurement conditions were summarized as follows.

Tester: tensilon-type tensile tester
Test Piece: 6 mm in width, 100 mm in length
Grip Distance: 25 mm
Crosshead Moving Speed (Tensile Speed): 300 mm/minute
Number of Repeated Tests: n = 3
Measurement Atmosphere: 23°C, 50% RH

[Moisture Permeability Measurement]

The moisture permeability was evaluated as the amount of water vapor that passes through a test material (a film-like material) of a unit area in a certain period of time. Specifically, under the atmosphere of 40°C, by setting a relative humidity in a space on one side separated by the tape of the test piece to be 90%, and by keeping a space on the other side to be in a dry state by a desiccant, a mass (g) of the water vapor passing through the test piece in 24 hours was measured and converted into a value per 1 $m^2$ of the test material.

The measurement was performed according to the condition B of Japanese Industrial Standard Z-0208 with the following procedures. A circular test piece that has a diameter greater than an inner diameter of a cup by about 10 mm was covered on the cup where a calcium chloride desiccant of about 15 g was introduced, and further, a rubber seal and a ring were covered and screwed in such a manner that the test piece was not displaced off.

After measuring the total mass of the test piece, the test piece was placed in a thermo-hygrostat bath under the atmosphere of 40°C and 90% RH. Mass variation was then measured at regular time intervals, and the moisture permeability was obtained by the following formula.

$$\text{Moisture permeability (g/m}^2 \cdot 24 \text{ h)} = W \times 24000/S$$

[In the formula, S indicates a moisture permeable area (cm$^2$), and W indicates a mass increase (g/h) per hour.]

The moisture permeability of the tape of the present invention is preferably equal to or more than 1,000 (g/m$^2 \cdot$24h). The tape having the moisture permeability equal to or more than 1,000 (g/m$^2 \cdot$24h) reduces stuffiness when the tape is attached on the skin so as to be less likely to cause the skin irritancy and an itch during attached, thus allowing attachment over a long period of time. The moisture permeability of the tape is more preferably equal to or more than 2,000 (g/m$^2 \cdot$24h), and further preferably equal to or more than 3,000 (g/m$^2 \cdot$24h). The higher moisture permeability, the more preferable result it would be possible to gain. However, although there is no upper limit for the preferable moisture permeability, the preferred moisture permeability is normally equal to or less than 30,000 (g/m$^2 \cdot$24h).

[Embodiment 1]

An ethyl acetate solution (total solid content is about 40 mass%) of the acrylic adhesive [obtained by reacting (2-ethylhexyl acrylate/vinyl acetate/acrylic acid: mass ratio of 85/11/4) of 100 pts.mass with epoxide-based resin (TETRAD-X, manufactured by MITSUBISHI GAS CHEMICAL COMPANY, INC.) of 0.04 pts.mass] was applied over one surface of the separation body so as to have the thickness of 40 g/m$^2$ after drying. Subsequently, the ethyl acetate solution was dried, thus forming the adhesion layer.

On the other hand, an ethylene-vinyl acetate copolymer (EVA) was applied over a surface on one side of the backing made of a polyester nonwoven fabric (basis weight 30 g/m$^2$), thus forming a plurality of the resin stripes of the ethylene-vinyl acetate copolymer (this resin stripe had the width of 0.5 mm, the pitch interval between these resin stripes was a regular interval, specifically 0.5 mm, these resin stripes formed a linear striped pattern, and an application amount of the resin stripe was 20 g/m$^2$ per area of the resin stripe).

Then, as illustrated in Fig. 2, the backing was laminated over the separation body such that the resin stripes were disposed on the adhesion layer side of the backing, thus manufacturing the tape.

[Embodiment 2 and Embodiment 3]

The tapes of the Embodiment 2 and the Embodiment 3 were manufactured by a method similar to the embodiment 1 other than the respective application amounts of the resin stripe changed to amounts described in Table 1.

[Comparative Example 1 to Comparative Example 4]

The tapes of the comparative example 1 to the comparative example 4 were each manufactured by a method similar to the embodiment 1 except that the resin stripe was not disposed on the backing and the backing was changed to a backing described in Table 1.

A PET nonwoven fabric in the comparative example 1 is identical to the base material of the embodiment 1 except that the resin stripe is not disposed.

[Table 1]

| Evaluation Item | Unit | No | Embodiment 1 | Embodiment 2 | Embodiment 3 | Comparative Example 1 | Comparative Example 2 | Comparative Example 3 | Comparative Example 4 |
|---|---|---|---|---|---|---|---|---|---|
| | | Backing | PET Nonwoven Fabric | PET Nonwoven Fabric | PET Nonwoven Fabric | PET Nonwoven Fabric | Rayon/Polyester Nonwoven Fabric | Rayon Nonwoven Fabric | PP Spun-bonded Nonwoven Fabric |
| | | Resin Stripe | EVA(20 g/m$^2$) | EVA(25 g/m$^2$) | EVA(30 g/m$^2$) | - | - | - | - |
| | | Adhesive | Acrylic Adhesive | Acrylic Adhesive | Acrylic Adhesive | Acrylic Adhesive | Acrylic Adhesive | Acrylic Adhesive | Acrylic Adhesive |
| Adhesive Force to YUPO | N/6 mm (MD) | | 1.6 | 1.6 | 1.8 | 1.5 | 2.1 | 1.6 | 1.3 |
| Self-Backside Adhesive Force | N/6 mm (MD, MD) | | 4.0 | 4.0 | 4.3 | 4.2 | 2.0 | 3.3 | 1.3 |
| [MD] 5%-Tensile-Load | N/6 mm (Chuck 25 mm) | | 6.1 | 6.7 | 7.3 | 3.3 | 1.8 | 3.0 | 1.8 |
| [MD] Maximum Point Load | N/6 mm (Chuck 25 mm) | | 21.6 | 21.0 | 21.4 | 18.5 | 17.5 | 9.6 | 5.8 |
| [MD] Elongation under Load of 2 N | %GL (6 mm Width, 25 mm Chuck) | | 1.9 | 1.7 | 1.4 | 3.1 | 1.2 | 3.0 | 6.0 |
| [MD] Elongation under Load of 3 N | %GL (6 mm Width, 25 mm Chuck) | | 2.6 | 2.4 | 2.0 | 4.7 | 1.7 | 5.0 | 13.0 |
| [CD] 5%-Tensile-Load | N/6 mm (Chuck 25 mm) | | 0.6 | 0.6 | 0.6 | 0.4 | 3.5 | 0.4 | 1.3 |

| Evaluation Item | Unit | No | Embodiment 1 | Embodiment 2 | Embodiment 3 | Comparative Example 1 | Comparative Example 2 | Comparative Example 3 | Comparative Example 4 |
|---|---|---|---|---|---|---|---|---|---|
| | | Backing | PET Nonwoven Fabric | PET Nonwoven Fabric | PET Nonwoven Fabric | PET Nonwoven Fabric | Rayon/Polyester Nonwoven Fabric | Rayon Nonwoven Fabric | PP Spun-bonded Nonwoven Fabric |
| | | Resin Stripe | EVA(20 g/m$^2$) | EVA(25 g/m$^2$) | EVA(30 g/m$^2$) | - | - | - | - |
| | | Adhesive | Acrylic Adhesive | Acrylic Adhesive | Acrylic Adhesive | Acrylic Adhesive | Acrylic Adhesive | Acrylic Adhesive | Acrylic Adhesive |
| [CD] Maximum Point Load | N/6 mm (Chuck 25 mm) | | 3.9 | 3.6 | 3.7 | 3.3 | 4.8 | 2.6 | 6.2 |
| [CD] Elongation under Load of 2 N | %GL (6 mm Width, 25 mm Chuck) | | 56.8 | 50.0 | 43.6 | 73.4 | 2.4 | 66.1 | 10.3 |
| [CD] Elongation under Load of 3 N | %GL (6 mm Width, 25 mm Chuck) | | 91.4 | 84.3 | 81.3 | 106.7 | 3.9 | - | 22.4 |
| Moisture Permeability | g/m$^2$·24h | | 3684 | 3377 | 3823 | 6275 | 2740 | 5307 | 5595 |

EP 3 326 593 A1

According to the result of Table 1, the tapes of the Embodiment 1 to the Embodiment 3, where a plurality of the resin stripes extending in the longitudinal direction were disposed on the surface of the backing made of the PET nonwoven fabric, have difficulty in extending in the longitudinal direction. Accordingly, it can ensure fixing the joined or sutured wound part so as not to open. Further, since the tapes are allowed to extend in the shorter side direction, it can provide excellent skin followability and excellent moisture permeability, thus allowing an effect of low skin irritation. The tapes of the Embodiment 1 to the embodiment 3 were attached on the skins of forearms of respective 20 examinees for 48 hours, and after 48 hours, a ratio of an area of the tape that attached to the skin to an area of the adhesion layer of the tape was calculated for each examinee. The average value of the ratios of the attached area of all the examinees was then calculated as an attachment rate. As a result, the attachment rate after 48 hours was as high as 97% even compared with the beginning of attachment (100%), and no skin irritancy was seen.

In contrast, the tape of the comparative example 1, where the resin stripe was not disposed on the surface of the backing made of the PET nonwoven fabric, showed a result that the tape was much extended both in the longitudinal direction and the shorter side direction compared with the tapes of the embodiment 1 to the embodiment 3.

The tape of the comparative example 2, where the backing made of a rayon/polyester nonwoven fabric was used, showed a result that the tape was less extended in the shorter side direction compared with the tapes of the embodiment 1 to the embodiment 3, thus causing an inferior self-backside adhesive force.

The tape of the comparative example 3, where the backing made of a rayon nonwoven fabric was used, showed a result that the tape was much extended in the longitudinal direction compared with the tapes of the embodiment 1 to the embodiment 3.

The tape of the comparative example 4, where the backing made of a PP spun-bonded nonwoven fabric was used, showed a result that the tape was much extended in the longitudinal direction compared with the tapes of the embodiment 1 to the embodiment 3, thus causing a significantly inferior self-backside adhesive force.

Reference Signs List

1    skin closure tape or reinforcing tape used after skin-suture
2    backing
3    resin stripe
4    adhesion layer

**Claims**

1.  A skin closure tape or a reinforcing tape used after skin-suture, comprising:

    a backing made of at least a nonwoven fabric, and
    an adhesion layer disposed on a surface on one side of the backing, wherein
    a plurality of resin stripes are disposed on a surface on another side or/and the surface on the one side of the backing, the plurality of the resin stripes extending in a longitudinal direction of the backing.

2.  The tape according to claim 1, wherein
    the plurality of the resin stripes are stripes that have constant widths and are regularly arranged on the surface of the backing.

3.  The tape according to claim 1 or claim 2, wherein
    the plurality of the resin stripes have equal pitch intervals.

4.  The tape according to claim 3, wherein
    the pitch interval of the plurality of the resin stripes is 0.1 mm to 4.0 mm.

5.  The tape according to any one of claim 1 to claim 4, wherein
    the resin stripes form a linear or approximately linear striped pattern.

6.  The tape according to any one of claim 1 to claim 5, wherein
    the nonwoven fabric is a polyester nonwoven fabric.

7.  The tape according to any one of claim 1 to claim 6, wherein
    the nonwoven fabric has a basis weight of 10 g/m$^2$ to 100 g/m$^2$.

8.  The tape according to any one of claim 1 to claim 7, wherein
    a self-backside adhesive force is 3.0 N/6 mm to 4.5 N/6 mm in a self-backside adhesive force test where an adhesive force is to be measured in that the adhesion layer of the tape is attached on a surface of a backing of a tape different from the tape.

9.  The tape according to any one of claim 1 to claim 8, wherein
    the resin stripe is a resin stripe made of an ethylene-vinyl acetate copolymer.

10. The tape according to any one of claim 1 to claim 9, wherein
    an application amount of the resin stripe is an amount of 10 g/m$^2$ to 100 g/m$^2$ per area of the resin stripe.

11. The tape according to any one of claim 1 to claim 10, wherein
    the tape has a 5%-tensile-load in a longitudinal direction of 4.0 N/6 mm to 15.0 N/6 mm and a 5%-tensile-load in a shorter side direction of 0.1 N/6 mm to 3.0 N/6 mm.

12. The tape according to any one of claim 1 to claim 11, wherein
    the resin stripe is disposed on an adhesion layer side of the backing.

F I G . 1

F I G . 2

FIG. 3

FIG. 4

**INTERNATIONAL SEARCH REPORT**

| International application No. |
| --- |
| PCT/JP2016/071398 |

A. CLASSIFICATION OF SUBJECT MATTER
*A61F13/02*(2006.01)i, *A61L17/00*(2006.01)i

According to International Patent Classification (IPC) or to both national classification and IPC

B. FIELDS SEARCHED

Minimum documentation searched (classification system followed by classification symbols)
A61F13/00-13/14, 15/00-17/00

Documentation searched other than minimum documentation to the extent that such documents are included in the fields searched
Jitsuyo Shinan Koho          1922-1996   Jitsuyo Shinan Toroku Koho   1996-2016
Kokai Jitsuyo Shinan Koho    1971-2016   Toroku Jitsuyo Shinan Koho   1994-2016

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | Microfilm of the specification and drawings annexed to the request of Japanese Utility Model Application No. 138728/1988(Laid-open No. 61933/1990) (Nitto Denko Corp.), 09 May 1990 (09.05.1990), (Family: none) | 1-12 |
| A | JP 3-501104 A  (Conmed, Inc.), 14 March 1991 (14.03.1991), & US 4965126 A          & WO 1988/008787 A1 | 1-12 |
| A | JP 2015-97792 A  (KCI Licensing, Inc.), 28 May 2015 (28.05.2015), & US 2010/0121286 A1    & WO 2010/053870 A1 & CA 2742962 A          & CN 102202619 A | 1-12 |

| ☒ Further documents are listed in the continuation of Box C. | ☐ See patent family annex. |
| --- | --- |

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| 06 October 2016 (06.10.16) | 18 October 2016 (18.10.16) |

| Name and mailing address of the ISA/ Japan Patent Office 3-4-3,Kasumigaseki,Chiyoda-ku, Tokyo 100-8915,Japan | Authorized officer

Telephone No. |
| --- | --- |

Form PCT/ISA/210 (second sheet) (January 2015)

**INTERNATIONAL SEARCH REPORT**

International application No.

PCT/JP2016/071398

C (Continuation). DOCUMENTS CONSIDERED TO BE RELEVANT

| Category* | Citation of document, with indication, where appropriate, of the relevant passages | Relevant to claim No. |
|---|---|---|
| A | US 6074965 A (BEIERSDORF AG), 13 June 2000 (13.06.2000), & EP 761187 A1 & DE 19531291 A1 | 1-12 |
| A | US 2006/0265005 A1 (BEESE, Stephen A.), 23 November 2006 (23.11.2006), & WO 2006/127385 A2 | 1-12 |
| P,A | JP 2016-69454 A (Sibel Sangyo Co., Ltd.), 09 May 2016 (09.05.2016), & JP 5872657 B1 | 1-12 |

Form PCT/ISA/210 (continuation of second sheet) (January 2015)

**EP 3 326 593 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 62243557 A
- JP 2004033265 A